# EUROPEAN PATENT APPLICATION

(11) **EP 3 103 400 A1**
(43) Date of publication of application: **14.12.2016**
(21) Application number: 16173411.6
(22) Date of filing: 07.06.2016
(51) Int. Cl.: A61B 17/072, A61B 90/30

(54) **SURGICAL INSTRUMENT WITH INTEGRATED ILLUMINATION**

(30) Priority: 08.06.2015 US 201514733166
(71) Applicant: Covidien LP, Mansfield, MA 02048 (US)
(72) Inventor: Schmitt, Jeffrey, Trumbull, CT 06611 (US)
(74) Representative: Soames, Candida Jane

(57) **Abstract**

A loading unit includes an elongated body, an end effector, and a lighting unit. The elongated body defines a longitudinal axis and has a distal end portion. The end effector is supported on the distal end portion of the elongated body. The lighting unit is disposed in the distal end portion of the elongated body and has a distal surface that is configured to emit light about the end effector to provide illumination of the end effector and an area distal to the end effector.

## Description

### BACKGROUND

### 1. Technical Field

The present disclosure relates to surgical instruments and, more specifically, to integrated illumination for surgical instruments.

### 2. Discussion of Related Art

During minimally invasive surgical procedures surgical instruments are inserted through openings or incisions to access a surgical site disposed within a body cavity. During such a surgical procedure, it is necessary to provide visualization of the surgical site. The surgical site may be illuminated to assist in visualization of the surgical procedure.

Generally, an access device is inserted through one opening in the body cavity to provide access for a surgical instrument and another access device is inserted through another opening to provide visualization of the surgical site. In addition, one or more additional access ports are inserted into additional openings to illuminate the surgical site or provide additional instrumentation. Each opening creates additional trauma to a patient.

There is a continuing need for surgical instruments for and methods of illuminating a surgical site within a body cavity that reduces the trauma to a patient.

### SUMMARY

In an aspect of the present disclosure, a loading unit includes an elongated body, an end effector, and a lighting unit. The elongated body defines a longitudinal axis and has a distal end portion. The end effector is supported on the distal end portion of the elongated body. The lighting unit is disposed in the distal end portion of the elongated body and has a distal surface that is configured to emit light about the end effector to provide illumination of the end effector and an area distal to the end effector. The distal surface may be in the shape of a ring about the longitudinal axis of the elongated body. The end effector may include first and second jaw members where one of the jaw members is moveable relative to the other one of the jaw members.

In aspects, the lighting unit includes a light source that is configured to generate light which is emitted through the distal surface. The light source may generate light by electron-stimulation, incandescent lamps, light emitting diodes, electroluminescence, gas discharge, high-intensity discharge, laser, chemoluminescence, fluorescence, phosphorescence, or any combination thereof.

In some aspects, the light source is configured to generate light having a specific wavelength to enhance visualization of the surgical site. The specific wavelength may be selectively adjustable during a surgical procedure. The distal surface may be configured to diffuse light generated by the light source. Additionally or alternatively, the distal surface may be configured to shape light emitted therethrough.

In another aspect of the present disclosure, a surgical instrument includes a handle assembly, an elongated portion, and a loading unit. The elongated portion extends from the handle assembly. The loading unit is supported at a distal end of the elongated portion and includes an elongated body, an end effector, and a lighting unit. The elongated body defines a longitudinal axis and has a distal end portion. The end effector is supported on the distal end portion of the elongated body. The lighting unit is disposed in the distal end portion of the elongated body. The lighting unit has a distal surface that is configured to emit light about the end effector to illuminate a surgical site distal to the end effector.

In aspects, the surgical instrument includes a light source that is configured to generate light which is emitted through the distal surface. The light source may be disposed within the lighting unit, the elongated body, or the handle assembly.

In another aspect of the present disclosure, a method of illuminating a surgical site includes positioning an end effector of a surgical instrument adjacent the surgical site and activating a light source of the surgical instrument to illuminate the surgical site. The light source generates light which is projected through a distal surface proximal to the end effector.

In aspects, the method includes treating tissue at the surgical site with the end effector of the surgical instrument. Positioning the end effector adjacent the surgical site may include inserting the end effector through an opening in a body cavity of a patient.

In some aspects, the light source generates light having a specific wavelength to enhance visualization of the surgical site. The method may include injecting a dye into vasculature of the patient or into tissue having vasculature. The dye may be configured to reflect or absorb a predetermined wavelength of light. The method may include adjusting the specific wavelength to the predetermined wavelength of light. Adjusting the specific wavelength may occur during a surgical procedure after the end effector is positioned adjacent the surgical site.

Further, to the extent consistent, any of the aspects described herein may be used in conjunction with any or all of the other aspects described herein.

### BRIEF DESCRIPTION OF THE DRAWINGS

Various aspects of the present disclosure are described hereinbelow with reference to the drawings, which are incorporated in and constitute a part of this specification, wherein:
FIG. 1 is a perspective view of a surgical instrument having a loading unit including a lighting unit in accordance with the present disclosure;
FIG. 2 is an enlarged view of the indicated area of detail of FIG. 1; and
FIG. 3 is a cut-away view of a body cavity of a patient with the surgical instrument of FIG. 1 inserted through an opening in a wall of the body cavity to access a surgical site.

### DETAILED DESCRIPTION

Embodiments of the present disclosure are now described in detail with reference to the drawings in which like reference numerals designate identical or corresponding elements in each of the several views. As used herein, the term "clinician" refers to a doctor, a nurse, or any other care provider and may include support personnel. Throughout this description, the term "proximal" refers to the portion of the device or component thereof that is closest to the clinician and the term "distal" refers to the portion of the device or component thereof that is farthest from the clinician.

This disclosure relates generally to surgical instruments including a lighting unit for enhancing visualization of a surgical site during a procedure. The lighting unit is positioned adjacent an end effector and is configured to illuminate a surgical site during the procedure. The lighting unit includes a distal ring having a distal surface that projects light generated by a light source to illuminate the surgical site. The light may have a specific wavelength to enhance visualization of desired structures within the surgical site. The wavelength of the light may be selectively adjustable by a clinician.

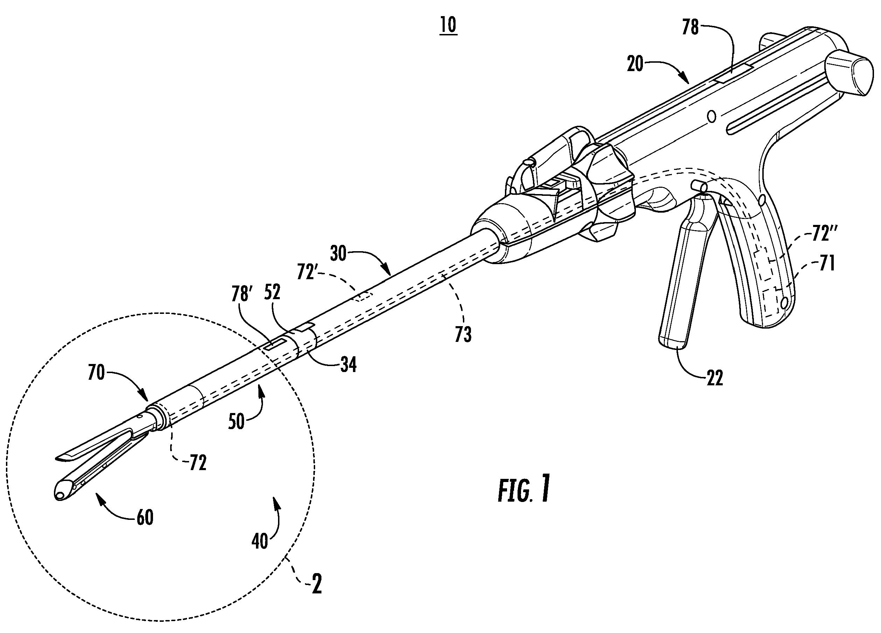

Referring now to FIG. 1, a surgical instrument 10 including a loading unit 40 is provided in accordance with the present disclosure. The surgical instrument 10 includes a handle assembly 20 and the loading unit 40. The handle assembly 20 includes a distally extending elongated portion 30. The loading unit 40 is disposed at a distal end 34 of the elongated portion 30 and includes an elongated body 50, a tool assembly or end effector 60, and a lighting unit 70. The handle assembly 20 is configured to actuate the end effector 60 of the loading unit 40 and to activate the lighting unit 70 of the loading unit 40.

The elongated body 50 has a proximal end 52 that is releasably coupled to the distal end 34 of the elongated portion 30. Alternatively, the elongated body 50 may be integrally formed with the elongated portion 30.

The handle assembly 20 is configured to actuate the end effector 60. Specifically, a moveable handle 22 of the handle assembly 20 is actuateable to advance a drive rod (not shown) which initially approximates jaws of the end effector 60. Additional advancement of the drive rod actuates the end effector 60 (e.g., fire staples, advance a knife, etc.).

For a detailed description of the structure and function of an exemplary handle assembly and loading unit, please refer to commonly owned U.S. Patent No. 7,565,993, which is incorporated herein by reference in its entirety.

As shown, the handle assembly 20 is a manually actuated handle assembly; however, it is contemplated that the handle assembly 20 may be an electromechianical handle assembly. Further, such an electromechanical handle assembly may include an adapter that couples to the electromechanical handle assembly and supports the loading unit 40. For a detailed description of the structure and function of an exemplary electromechanical handle assembly and adapter, please refer to commonly owned U.S. Patent Publication No. 2012/0253329 and U.S. Patent No. 8,968,276. Each of these disclosures is incorporated herein by reference in its entirety. Furthermore, the instrument may be part of, or configured to be used with, a robotic surgical system.

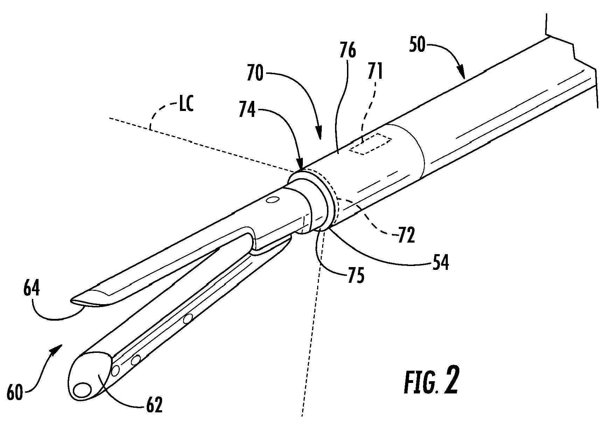

Referring to FIG. 2, the elongated body 50 also has a distal end 54 that supports the end effector 60. The end effector 60 includes first and second jaw members 62, 64 that are moveable relative to one another. The first and second jaw members 62, 64 may include surgical fasteners for joining tissue together, may include gripping surfaces for manipulating tissue, may include a knife for severing tissue, may include sealing plates for delivering electrosurgical energy to tissue, or may include any combination thereof. It is also contemplated that the end effector 60 may include a single member (not explicitly shown) to treat tissue.

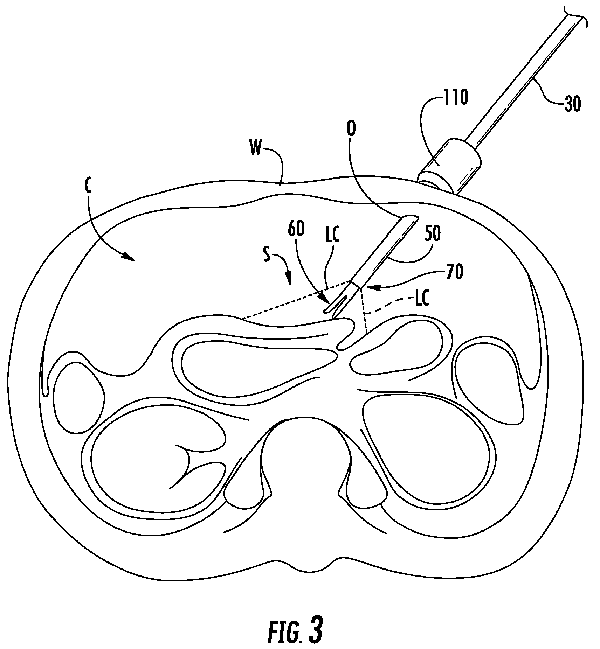

With additional reference to FIG. 3, the lighting unit 70 is configured to illuminate a surgical site S. The lighting unit 70 is disposed adjacent the distal end 54 of the elongated body 50. The lighting unit 70 includes a light source 72 and a distal ring 74 that is in optical communication with the light source 72 such that light generated by the light source 72 is emitted from the distal ring 74. The light source 72 may generate light any known means including, but not limited to, electron-stimulation, incandescent lamps, light emitting diodes, electroluminescence, gas discharge, high-intensity discharge, laser, chemoluminescence, fluorescence, phosphorescence, or a combination thereof.

It is also contemplated that the light source 72 may be located outside of the lighting unit 70. With particular reference to FIG. 1, a light source 72' may be positioned in the elongated portion 30 and/or a light source 72" may be disposed within the handle assembly 20. Light generated by the light sources 72, 72', 72" may travel through light pipes 73 positioned in or along the hand assembly 20, the elongated portion 30, and/or the elongated body 50 which optically connects the light sources 72, 72', 72" to the distal ring 74. The lighting unit 70 may also include an energy source 71 that selectively supplies energy to one or more of the light sources 72, 72', 72" which then convert the energy to light. As discussed below, the light sources 72, 72', 72" will be detailed with reference to the singular light source 72.

As shown, the energy source 71 is positioned within the lighting unit 70 adjacent the light source 72. It is contemplated that the energy source 71 may be positioned outside of the lighting unit 70 and may be positioned remote to the light source 72. For example, the energy source 71 may be positioned within the handle assembly 20, the elongated portion 30, the elongated body 50, or the lighting unit 70. The energy source 71 may supply electrical energy to the light source 72.

With particular reference to FIG. 1, the handle assembly 20 may include a switch 78 to selectively activate the light source 72 (i.e., operatively connect the energy source 71 to the light source 72). As shown in FIG. 1, the switch 78 is a slide switch; however, the switch 78 may be a toggle switch, a push button, etc. Additionally or alternatively, the loading unit 40 may include a switch 78' positioned on the elongated body 50 such that the lighting unit 70 may be contained and controlled entirely within the loading unit 40. It is contemplated that the switch 78 may be on moveable handle 22. Additionally or alternatively, the switch 78 may be positioned such that as the moveable handle 22 is actuated, the switch 78 is actuated to activate the light source 72.

With reference to FIGS. 2 and 3, the distal ring 74 has a distal surface 75 that is shaped to project a light cone LC from light generated by the light source 72 such that a surgical site S is illuminated. Additionally or alternatively, the distal surface 75 may be shaped to project a light cone LC from light generated by the light source 72 such that the end effector 60 is illuminated. As shown, the light source 72 has an annular shape which emits light about the entire distal ring 74; however, the light source 72 may generate a point source of light which is shaped by the distal ring 74 to project light from the entire distal surface 75. As shown, the distal surface 75 is substantially planar; however, the distal surface 75 may be angular, prismatic, convex, concave, or any combination thereof to project a desired light cone LC. The distal surface 75 may also inhibit light from emitting from portions of the distal ring 74. The distal surface 75 may also diffuse and/or filter light to enhance visualization of the surgical site.

The lighting unit 70 has an outer surface 76 that extends proximally from the distal ring 74. The outer surface 76 is substantially cylindrical in shape and has a diameter substantially equal to a diameter of the elongated body 50 of the loading unit 40. The outer surface 76 of the lighting unit 70 may also project light generated by the light source 72 to enhance visualization of the surgical site S.

In embodiments of the present disclosure, the light source 72 generates light of a specific wavelength to highlight types of tissue at the surgical site S. For example, the light source 72 may generate light having a wavelength that enhances visualization of vasculature at the surgical site S. The wavelength of light may be absorbed or reflected by the vasculature. Additionally, a dye may be used in conjunction with a specific wavelength of light to enhance the visualization of vasculature or a specific tissue at the surgical site S. It is also contemplated that the light source 72 may generate a spectrum of light and the distal surface 75 may filter the spectrum of light such that one or more specific wavelengths of light are projected from the distal surface 75.

With particular reference to FIG. 3, a method of illuminating a surgical site S in accordance with the present disclosure is disclosed. Initially, the end effector 60 is positioned adjacent a surgical site S. The end effector 60 and/or the elongated body 50 may be inserted through an opening O in a wall W defining a body cavity C. The opening O may be a natural opening or an incision in the wall W of the body cavity C. An access port 110 may be inserted in the opening and the end effector 60 may be inserted through a lumen of the access port 110. The method also includes activating the light source 72 to generate light which is projected through the distal surface 75 to form a light cone LC as detailed above. The light source 72 may be activated before or after the end effector 60 is positioned adjacent the surgical site S. As detailed above, the light source 72 may generate light that has a specific wavelength which enhances visualization of tissue of the surgical site. The method may also include injecting a dye into vasculature of the patient which is configured to reflect or absorb the specific wavelength of light. The light source 72 may be activated by actuating the switch 78 (FIG. 1).

While several embodiments of the disclosure have been shown in the drawings, it is not intended that the disclosure be limited thereto, as it is intended that the disclosure be as broad in scope as the art will allow and that the specification be read likewise. For example, the surgical instrument could be a number of different types of surgical instruments, such as a surgical stapler, or other types of instruments, such as electrosurgical which can incorporate the illumination feature described herein. Any combination of the above embodiments is also envisioned and is within the scope of the appended claims. Therefore, the above description should not be construed as limiting, but merely as exemplifications of particular embodiments. Those skilled in the art will envision other modifications within the scope of the claims appended hereto.

The invention may be described by reference to the following numbered paragraphs:-
1. A loading unit comprising:
   an elongated body defining a longitudinal axis and having a distal end portion;
   an end effector supported on the distal end portion of the elongated body; and
   a lighting unit disposed in the distal end portion of the elongated body, the lighting unit having a distal surface that is configured to emit light about the end effector to provide illumination of the end effector and an area distal of the end effector.
2. The loading unit according to paragraph 1, wherein the distal surface is in the shape of a ring about the longitudinal axis of the elongated body.
3. The loading unit according to paragraph 1, wherein the lighting unit includes a light source configured to generate light which is emitted through the distal surface.
4. The loading unit according to paragraph 3, wherein the light source generates light by at least one of electron-stimulation, incandescent lamps, light emitting diodes, electroluminescence, gas discharge, high-intensity discharge, laser, chemoluminescence, fluorescence, phosphorescence, or a combination thereof.
5. The loading unit according to paragraph 3, wherein the light source is configured to generate light having a specific wavelength to enhance visualization of the surgical site.
6. The loading unit according to paragraph 5, wherein a specific wavelength is selectively adjustable.
7. The loading unit according to paragraph 3, wherein the distal surface is configured to diffuse light generated by the light source.
8. The loading unit according to paragraph 3, wherein the distal surface is configured to shape light emitted therethrough.
9. The loading unit according to paragraph 1, wherein the end effector includes a first jaw member and a second jaw member, one of the jaw members being movable relative to the other one of the jaw members.
10. A surgical instrument comprising:
   a handle assembly;
   an elongated portion extending from the handle assembly; and
   a loading unit supported at a distal end of the elongated portion, the loading unit including:
      an elongated body defining a longitudinal axis and having a distal end portion;
      an end effector supported on the distal end portion of the elongated body; and
      a lighting unit disposed in the distal end portion of the elongated body, the lighting unit having a distal surface that is configured to emit light about the end effector to illuminate a surgical site.
11. The surgical instrument according to paragraph 10, further comprising a light source configured to generate light which is emitted through the distal surface.
12. The surgical instrument according to paragraph 11, wherein the light source is disposed in the lighting unit.
13. The surgical instrument according to paragraph 11, wherein the light source is disposed in the elongated body.
14. The surgical instrument according to paragraph 11, wherein the light source is disposed in the handle assembly.
15. A method of illuminating a surgical site, the method comprising:
   positioning an end effector of a surgical instrument adjacent the surgical site; and
   activating a light source of the surgical instrument to illuminate the surgical site, the light source generating light which is projected through a distal surface proximal to the end effector.
16. The method according to paragraph 15, further comprising treating tissue at the surgical site with the end effector of the surgical instrument.
17. The method according to paragraph 15, wherein positioning the end effector adjacent the surgical site includes inserting the end effector through an opening in a body cavity of a patient.
18. The method according to paragraph 14, wherein the light source generates light having a specific wavelength to enhance visualization of the surgical site.
19. The method according to paragraph 18, further comprising injecting a dye into tissue of the patient, the dye configured to reflect or absorb a predetermined wavelength of light.
20. The method according to paragraph 19, further comprising adjusting the specific wavelength to the predetermined wavelength of light.

## Claims

1. A loading unit comprising:
an elongated body defining a longitudinal axis and having a distal end portion;
an end effector supported on the distal end portion of the elongated body; and
a lighting unit disposed in the distal end portion of the elongated body, the lighting unit having a distal surface that is configured to emit light about the end effector to provide illumination of the end effector and an area distal of the end effector.

2. The loading unit according to claim 1, wherein the distal surface is in the shape of a ring about the longitudinal axis of the elongated body.

3. The loading unit according to claim 1 or claim 2, wherein the lighting unit includes a light source configured to generate light which is emitted through the distal surface; preferably wherein the light source generates light by at least one of electron-stimulation, incandescent lamps, light emitting diodes, electroluminescence, gas discharge, high-intensity discharge, laser, chemoluminescence, fluorescence, phosphorescence, or a combination thereof.

4. The loading unit according to claim 3, wherein the light source is configured to generate light having a specific wavelength to enhance visualization of the surgical site; preferably wherein a specific wavelength is selectively adjustable.

5. The loading unit according to claim 3, wherein the distal surface is configured to diffuse light generated by the light source; and/or wherein the distal surface is configured to shape light emitted therethrough.

6. The loading unit according to any preceding claim, wherein the end effector includes a first jaw member and a second jaw member, one of the jaw members being movable relative to the other one of the jaw members.

7. A surgical instrument comprising:
a handle assembly;
an elongated portion extending from the handle assembly; and
a loading unit supported at a distal end of the elongated portion, the loading unit including:
an elongated body defining a longitudinal axis and having a distal end portion;
an end effector supported on the distal end portion of the elongated body; and
a lighting unit disposed in the distal end portion of the elongated body, the lighting unit having a distal surface that is configured to emit light about the end effector to illuminate a surgical site.

8. The surgical instrument according to claim 7, further comprising a light source configured to generate light which is emitted through the distal surface.

9. The surgical instrument according to claim 7 or claim 8, wherein the light source is disposed in the lighting unit; and/or wherein the light source is disposed in the elongated body; and/or wherein the light source is disposed in the handle assembly.

10. A method of illuminating a surgical site, the method comprising:
positioning an end effector of a surgical instrument adjacent the surgical site; and
activating a light source of the surgical instrument to illuminate the surgical site, the light source generating light which is projected through a distal surface proximal to the end effector.

11. The method according to claim 10, further comprising treating tissue at the surgical site with the end effector of the surgical instrument.

12. The method according to claim 10 or claim 11, wherein positioning the end effector adjacent the surgical site includes inserting the end effector through an opening in a body cavity of a patient.

13. The method according to any of claims 10 to 12, wherein the light source generates light having a specific wavelength to enhance visualization of the surgical site.

14. The method according to claim 13, further comprising injecting a dye into tissue of the patient, the dye configured to reflect or absorb a predetermined wavelength of light.

15. The method according to claim 14, further comprising adjusting the specific wavelength to the predetermined wavelength of light.
